Europäisches Patentamt

European Patent Office

Office européen des brevets

(11) Publication number: **0 226 679 B1**

# EUROPEAN PATENT SPECIFICATION

(45) Date of publication of patent specification: **28.08.91** (51) Int. Cl.⁵: **A61K 31/565**

(21) Application number: **85309500.8**

(22) Date of filing: **24.12.85**

The file contains technical information submitted after the application was filed and not included in this specification

(54) **Contraceptive method and kit.**

(43) Date of publication of application:
**01.07.87 Bulletin 87/27**

(45) Publication of the grant of the patent:
**28.08.91 Bulletin 91/35**

(84) Designated Contracting States:
**AT BE CH DE FR GB IT LI LU NL SE**

(56) References cited:
**DE-A- 2 431 704**

(73) Proprietor: **WARNER-LAMBERT COMPANY**
**201 Tabor Road**
**Morris Plains New Jersey 07950(US)**

(72) Inventor: **Boissonneault, Roger**
**38 Jackie Drive**
**Long Valley New Jersey 07853(US)**

(74) Representative: **Jones, Michael Raymond et al**
**HASELTINE LAKE & CO. Hazlitt House 28**
**Southampton Buildings Chancery Lane**
**London WC2A 1AT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The study of the prevention of pregnancy in human females has led to the evolution of many hormone-based compositions. Some compositions contain both estrogenic and progestogenic compounds. Such compositions, referred to herein as "estrogen/progestogen combinations", are highly effective in controlling ovulation and conception.

Many estrogen/progestogen combination formulations suffer from the drawback that they induce, in a significant number of females, unwanted side effects, such as breakthrough bleeding or spotting. Applicant has discovered that such side effects can be minimized via the concomitant use of (1) particular estrogen/progestogen combinations and (2) administration of those combinations at appropriately-timed intervals.

It has been discovered that the unwanted side effects generally associated with the administration of estrogen/progesterone contraceptive schemes can be minimized when the compositions used correspond to phases containing, in sequence, 0.5-1.5 mcg norethindrone acetate and 10.50 mcg ethinyl estradiol for the first phase (Phase I); 0.5-1.5 mg norethindrone acetate and 10.50 mcg ethinyl estradiol in the second phase (Phase II); 0.5-1.5 mg norethindrone acetate and 10-50 mcg ethinyl estradiol in the third phase (Phase III) and a suitable quantity of an iron supplement, e.g., ferrous fumarate, or other non-steroidal agent or placebo in an optional fourth, or inactive, phase (Phase IV). It is essential that the phases succeed each other in increasing numeric order (i.e., I, II, III, IV).

Applicant's combinations can be characterized in that the amount of estrogen employed in any two phases is never the same. That is, the amount of ethinyl estradiol or other estrogenic component is different for each composition with different compositions being used in each phase.

U.S. Patent 4,390,531 uses norethindrone as the progestogenic component in one type of estrogen/protestogen combination. However, such combinations are generally associated with high incidence of breakthrough bleeding. Furthermore, Applicant employs a highly active progestogenic compound, eliminating the need for large amounts of this component.

Applicant's four-phase system can be administered in 23- to 34-day cycles, with 21- to 28-day cycles preferred. Generally, the first phase will be 4 to 7 days; the second will be 5 to 8 days; the third will be 7 to 12 days; and the fourth, or last, will be 7 days.

The invention has several advantages over prior art methods which employ estrogen/progestogen combinations. Principal among its advantages are ease of administration and similarity to the natural menstrual cycle -- i.e., similarity to cycles experienced by females who do not use contraceptive drugs.

In addition, use of the invention will potentially produce fewer side effects, most particularly, breakthrough bleeding. The compositions used herein preferably contain norethindrone acetate, a progestogenic component having less potency - and, concomitantly, producing lower incidence of breakthrough bleeding - than other progestogenic agents, e.g., norethindrone.

Other advantages and aspects of the invention will be apparent from a consideration of the following description.

The invention covers a method and kit for the use of a multiphase, sequentially-administered combination of compositions.

In one aspect, the invention deals with a method of contraception comprising the steps of sequentially administering, to a female of child bearing age:

(1) for 4 to 7 days, a composition I containing norethindrone acetate and ethinyl estradiol,

(2) for 5 to 8 days, a composition II containing norethindrone acetate and ethinyl estradiol, and

(3) for 7 to 12 days, a composition III containing norethindrone acetate and ethinyl estradiol;

wherein compositions I, II and III each contain from 0.5 to 1.5 mg norethindrone acetate and 10 to 50 μg ethinyl estradiol, and wherein the amount of ethinyl estradiol is increased stepwise over the three compositions I, II and III.

In another aspect, the invention is concerned with a multiphase combination and contraceptive kit comprising a package containing daily dosages of:

(1) a Phase I composition containing norethindrone acetate and ethinyl estradiol;

(2) a Phase II composition containing norethindrone acetate and ethinyl estradiol; and

(3) a Phase III composition containing norethindrone acetate and ethinyl estradiol.

An optional Phase IV composition, which contains an iron supplement, e.g., ferrous fumarate, and/or one or more placebos, can be used in conjunction with the other three.

The compositions are consumed or administered in a numeric sequence with the Phase I composition being used first, the Phase II composition being used second, etc. If packaging and/or other requirements dictate, the method and kit described herein can be employed as part of a larger scheme for contraception

2

or treatment of gynecological disorders. While the sequence in which Applicant's combinations are administered is important to their operation, it should be kept in mind that variations in timing and dosage can be tolerated when medical considerations so dictate.

The compositions employed in accordance with the invention in Phases I through IV will preferably have the administration times and drug contents set forth in the following table when a four-phase system is used. The table sets forth relevant values for one of our preferred embodiments, or configurations, for administration of the system to females.

Table

| Phase | Administration Interval, Days | Norethindrone Acetate, mg | Ethinyl Estradiol, mcg | Ferrous Fumarate, mg |
|-------|------------------------------|---------------------------|------------------------|----------------------|
| I | 5 | 1.0 | 20 | 0 |
| II | 7 | 1.5 | 30 | 0 |
| III | 9 | 1.0 | 50 | 0 |
| IV | 7 | 0 | 0 | 75 |

The norethindrone acetate used in the table is Norlutate®, a product manufactured by the Parke-Davis Division of Warner-Lambert Company, Morris Plains, New Jersey.

The designation "mcg" refers to micrograms and "mg" to milligrams.

It should be noted that this table is presented for illustrative purposes only. The substitution of functionally equivalent amounts and kinds of reagent(s) in these schemes is contemplated. For example, the use of sugar or other placebo in place of all or part of the ferrous fumarate is envisioned.

In addition, the use of other conventional additives, e.g., fillers, colorants and polymeric binders is also contemplated. In general any pharmaceutically-acceptable additive which does not interfere with the function of the active components can be used in one or more of the compositions.

Suitable carriers with which the compositions can be administered include lactose, starch and cellulose derivatives used in suitable amounts. Lactose is a preferred carrier. Mixtures of carriers are operable.

While the invention is discussed as one employing four phases, it may employ only three. Phase IV is not essential to the operation of the other three distinct phases. Thus a method or kit which does not contain the Phase IV component is operable and, in fact, will be preferred when suitable factors, e.g., economy, dictate the non-use of the Phase IV component.

The terms "method" and "kit" are used herein to encompass any drug delivery systems via the use of which the 3- or 4- phase scheme outlined above can be effectively administered to human females. Combinations of various dosage forms are operable.

As this description illustrates, applicant has discovered that a unique dosage pattern, i.e., a unique sequence of administrations of a novel estrogen/progestogen combination minimize certain side effects, notably breakthrough bleeding, commonly associated with conventional low dosage pills.

The administration sequence, i.e., the administration of the first pill or other dosage form containing the first dosage of composition 1, should take place from about 5 to 7 days after the start of menstruation (counting the first day of menstruation as day 1), preferably on or about the fifth day after the onset of menstruation. However, for ease of administration and to facilitate patient compliance, it is desirable to administer the first dosage of the first composition on or about the first Sunday after the menstrual period starts.

The use of the word "about" in relation to a range of days may mean that specific figures mentioned can be increased or decreased by up to twelve hours (e.g. 1, 2, 3, 4, 5, 6, 7, 8, 9, 10, 11 or 12 hours).

**Claims**

1.  A method of contraception comprising the steps of sequentially-administering to a female of child bearing age:

    (1) for 4 to 7 days, a composition I containing norethindrone acetate and ethinyl estradiol,

    (2) for 5 to 8 days, a composition II containing norethindrone acetate and ethinyl estradiol, and

    (3) for 7 to 12 days, a composition III containing norethindrone acetate and ethinyl estradiol;

    wherein compositions I, II and III each contain from 0.5 to 1.5 mg norethindrone acetate and 10 to 50 µg ethinyl estradiol, and wherein the amount of ethinyl estradiol is increased stepwise over the three compositions I, II and III.

2. The method of claim 1 which comprises the additional step of administering, for 7 days, a composition IV containing ferrous fumarate.

3. The method of claim 1 or 2, wherein composition I is administered for about 5 days and contains about 1.0 mg norethindrone acetate and about 20 µg ethinyl estradiol; composition II is administered for about 7 days and contains about 1.5 mg norethindrone acetate and about 30 µg ethinyl estradiol; and composition III is administered for about 9 days and contains about 1.0 mg norethindrone acetate and about 50 µg ethinyl estradiol.

4. The method of any preceding claim, wherein at least one composition is administered in combination with a suitable carrier.

5. A multiphase combination and contraceptive kit comprising a package containing daily dosages of:
   (1) a Phase I composition containing norethindrone acetate and ethinyl estradiol;
   (2) a Phase II composition containing norethindrone acetate and ethinyl estradiol; and
   (3) a Phase III composition containing norethindrone acetate and ethinyl estradiol;
   wherein the compositions for Phases I, II and III each contain in the range of from 0.5 to 1.5 mg norethindrone acetate, and from 10 to 50 µg ethinyl estradiol, and wherein the amount of ethinyl estradiol is increased stepwise over the three compsoitions I, II and III.

6. The kit of claim 5 which additionally comprises a Phase IV composition containing ferrous fumarate.

7. The kit of claim 6, wherein the composition for Phase IV contains about 75 mg ferrous fumarate.

8. The kit according to any of claims 5 to 7, containing about 5 dosages of the Phase I composition, about 6 dosages of the Phase II composition, and about 10 dosages of the Phase III composition.

9. The kit according to any of claims 5 to 7, wherein the Phase I composition contains about 1.0 mg norethindrone acetate and about 20 µg ethinyl estradiol; the Phase II composition contains about 1.5 mg norethindrone acetate and about 30 µg ethinyl estradiol; and the Phase III composition contains about 1.0 mg norethindrone acetate and about 50 µg ethinyl estradiol.

10. The kit of claim 9, containing about 5 dosages of the Phase I composition, about 7 dosages of the Phase II composition, and about 9 dosages of the Phase III composition.

11. The kit of claim 10 when appendant to claim 6 which additionally contains about 7 dosages of the Phase IV composition.

12. The kit of claim 8 when appendant to claim 6 which additionally contains about 7 dosages of the Phase IV composition.

**Revendications**

1. Une méthode de contraception comprenant les étapes d'administration séquentielle à une femme en âge de procréer:
   (1) pendant 4 à 7 jours, d'une composition I contenant de l'acétate de noréthindrone et de l'éthinyl-oestradiol,
   (2) pendant 5 à 8 jours, d'une composition II contenant de l'acétate de noréthindrone et de l'éthinyl-oestradiol, et
   (3) pendant 7 à 12 jours, d'une composition III contenant de l'acétate de noréthindrone et de l'éthinyl-oestradiol,
   lesdites compositions I, II et III contenant chacune de 0,5 à 1,5 mg d'acétate de noréthindrone et de 10 à 50 µg d'éthinyl-oestradiol, et les proportions d'éthinyl-oestradiol étant présentes dans chacune des trois compositions I, II et III en proportions respectivement croissantes.

2. La méthode selon la revendication 1, qui comprend l'étape supplémentaire d'administration, pendant 7 jours, d'une composition IV contenant du fumarate ferreux.

3. La méthode selon la revendication 1 ou 2, dans laquelle la composition I est administrée pendant environ 5 jours et contient environ 1,0 mg d'acétate de noréthindrone et environ 20 μm d'éthinyl-oestradiol; la composition II est administrée pendant environ 7 jours et contient environ 1,5 mg d'acétate de noréthindrone et environ 30 μm d'éthinyl-oestradiol; la composition III est administrée pendant environ 9 jours et contient environ 1,0 mg d'acétate de noréthindrone et environ 50 μg d'éthinyl-oestradiol.

4. La méthode selon l'une quelconque des revendications précédentes, dans laquelle au moins l'une des compositions est administrée en combinaison avec un support approprié.

5. Une trousse contraceptive à base de combinaison multiphase comprenant un conditionnement contenant des dosages journaliers de:
   (1) une composition de phase I contenant de l'acétate de noréthindrone et de l'éthinyl-oestradiol;
   (2) une composition de phase II contenant de l'acétate de noréthindrone et de l'éthinyl-oestradiol;
   (3) une composition de phase III contenant de l'acétate de noréthindrone et de l'éthinyl-oestradiol;
   lesdites compositions des phases I, II et III contenant chacune de 0,5 à 1,5 mg d'acétate de noréthindrone et de 10 à 50 μg d'éthinyl-oestradiol, et ladite proportion d'éthinyl-oestradiol étant présente en proportions croissantes dans chacune des trois compositions I, II et III respectivement.

6. La trousse selon la revendication 5, qui contient de plus une composition de phase IV contenant du fumarate ferreux.

7. La trousse selon la revendication 6, dans laquelle la composition de phase IV contient environ 75 mg de fumarate ferreux.

8. La trousse selon l'une quelconque des revendications 5 à 7, contenant environ 5 dosages de la composition de phase I, environ 6 dosages de la composition de phase II et environ 6 dosages de la composition de phase III.

9. La trousse selon l'une quelconque des revendications 5 à 7, dans laquelle la composition de phase I contient environ 1,0 mg d'acétate de noréthindrone et environ 20 μg d'éthinyl-oestradiol; la composition de phase II contient environ 1,5 mg d'acétate de noréthindrone et environ 30 μg d'éthinyl-oestradiol; et la composition de phase III contient environ 1,0 mg d'acétate de noréthindrone et environ 50 μg d'éthinyl-oestradiol.

10. La trousse selon la revendication 9, contenant environ 5 dosages de la composition de phase I, environ 7 dosages de la composition de phase II, et environ 9 dosages de la composition de phase III.

11. La trousse selon la revendication 10 en combinaison avec la revendication 6, qui contient de plus environ 7 dosages de la composition de phase IV.

12. La trousse selon la revendication 8 en combinaison avec la revendication 6, qui contient de plus environ 7 dosages de la composition de phase IV.

**Patentansprüche**

1. Verfahren zur Empfängnisverhütung, welches die Schritte der aufeinanderfolgenden Verabreichung:
   (1) einer Norethindronacetat und Ethinylöstradiol enthaltenden Zusammensetzung I während vier bis sieben Tagen,
   (2) einer Norethindronacetat und Ethinylöstradiol enthaltenden Zusammensetzung II während fünf bis acht Tagen und
   (3) einer Norethindronacetat und Ethinylöstradiol enthaltenden Zusammensetzung III während sieben bis zwölf Tagen an eine Frau im gebärfähigen Alter umfaßt,
   in welchem Verfahren die Zusammensetzungen I, II und III jeweils von 0,5 bis 1,5 mg Norethindronacetat und 10 bis 50 μg Ethinylöstradiol enthalten, und worin die Merge an Ethinylöstradiol schrittweise

5

über die drei Zusammensetzungen I, II und III erhöht wird.

2. Verfahren nach Anspruch 1, welches den zusätzlichen Schritt der Verabreichung einer Eisen(II)-fumarat enthaltenden Zusammensetzung IV während sieben Tagen umfaßt.

3. Verfahren nach Anspruch 1 oder 2, worin die Zusammensetzung I etwa fünf Tage lang verabreicht wird und etwa 1,0 mg Norethindronacetat und etwa 20 $\mu$g Ethinylöstradiol enthält, worin die Zusammensetzung II etwa sieben Tage lang verabreicht wird und etwa 1,5 mg Norethindronacetat und etwa 30 $\mu$g Ethinylöstradiol enthält, und worin die Zusammensetzung III etwa neun Tage lang verabreicht wird und etwa 1,0 mg Norethindronacetat und etwa 50 $\mu$g Ethinylöstradiol enthält.

4. Verfahren nach irgendeinem vorhergehenden Anspruch, worin zumindest eine Zusammensetzung zusammen mit einem geeigneten Träger verabreicht wird.

5. Multiphasenkombination und Empfängnisverhütungsausrüstung, welche eine Packung enthaltend Tagesdosen:
   (1) einer Norethindronacetat und Ethinylöstradiol enthaltenden Phase-I-Zusammensetzung,
   (2) einer Norethindronacetat und Ethinylöstradiol enthaltenden Phase-II-Zusammensetzung und
   (3) einer Norethindronacetat und Ethinylöstradiol enthaltenden Phase-III-Zusammensetzung,
   worin die Zusammensetzungen für die Phasen I, II und III jeweils Norethindronacetat im Bereich von 0,5 bis 1,5 mg und Ethinylöstradiol im Bereich von 10 bis 50 $\mu$g enthalten, und worin die Menge an Ethinylöstradiol schrittweise über die drei Zusammensetzungen I, II und III erhöht wird, umfaßt.

6. Ausrüstung nach Anspruch 5, welche zusätzlich eine Eisen(II)-fumarat enthaltende Phase-IV-Zusammensetzung umfaßt.

7. Ausrüstung nach Anspruch 6, worin die Zusammensetzung für die Phase IV etwa 75 mg Eisen(II)-fumarat enthält.

8. Ausrüstung nach irgendeinem der Ansprüche 5 bis 7, welche etwa fünf Dosierungen der Phase-I-Zusammensetzung, etwa sechs Dosierungen der Phase-II-Zusammensetzung und etwa zehn Dosierungen der Phase-III-Zusammensetzung enthält.

9. Ausrüstung nach irgendeinem der Ansprüche 5 bis 7, worin die Phase-I-Zusammensetzung etwa 1,0 mg Norethindronacetat und etwa 20 $\mu$g Ethinylöstradiol enthält, worin die Phase-II-Zusammensetzung etwa 1,5 mg Norethindronacetat und etwa 30 $\mu$g Ethinylöstradiol enthält; und die Phase-III-Zusammensetzung etwa 1,0 mg Norethindronacetat und etwa 50 $\mu$g Ethinylöstradiol enthält.

10. Ausrüstung nach Anspruch 9, welche etwa fünf Dosierungen der Phase-I-Zusammensetzung, etwa sieben Dosierungen der Phase-II-Zusammensetzung und etwa neun Dosierungen der Phase-III-Zusammensetzung enthält.

11. Ausrüstung nach Anspruch 10, wenn auf Anspruch 6 rückbezogen, welche zusätzlich etwa sieben Dosierungen der Phase-IV-Zusammensetzung enthält.

12. Ausrüstung nach Anspruch 8, wenn auf Anspruch 6 rückbezogen, welche zusätzlich etwa sieben Dosierungen der Phase-IV-Zusammensetzung enthält.